# EUROPÄISCHE PATENTSCHRIFT

(11) **EP 1 398 641 B1**
(45) Veröffentlichungstag und Bekanntmachung des Hinweises auf die Patenterteilung: **18.10.2006**
(21) Anmeldenummer: 02020475.6
(22) Anmeldetag: 12.09.2002
(51) Int. Cl.: G01R 33/28, A61M 5/168

(54) **Verteilungsbestimmung zur Infusionsplanung**
Determination of distributions for the planning of infusions
Détermination de distributions pour le planning d'infusions

(43) Veröffentlichungstag der Anmeldung: 17.03.2004
(73) Patentinhaber: BrainLAB AG, 85551 Kirchheim/Heimstetten (DE)
(72) Erfinder: Pedain, Christoph, 81667 München (DE); Hartlep, Andreas, Dr., 80803 München (DE); Raghavan, Raghu, Dr., Baltimore MD 21210 (US); Brady, Martin, Dr., 600 Wyndhurst Ave., Baltimore MD 21210 (US)
(74) Vertreter: Schwabe - Sandmair - Marx

(56) Entgegenhaltungen:
- EP-A- 0 702 966
- US-A- 5 205 289
- US-A- 5 583 902
- US-A- 6 061 587

## Beschreibung

Die Erfindung betrifft das Gebiet der Infusionsplanung. Es werden ein Verfahren zur Identifizierung von vorteilhaften bzw. nicht vorteilhaften Infusionsbereichen sowie ein Verfahren und eine Vorrichtung zur Planungsunterstützung für die Einbringung eines Infusionsfluids offenbart.

Verschiedene medizinische Verfahren erfordern es, therapeutische Mittel direkt in das Gewebe zu infundieren, mit dem Ziel, eine breite und bestmögliche Homogenität der Verteilung des Infusionsfluids im Gewebe zu erreichen. Es wird sich bei dem verabreichten Mittel meist um Fluide handeln, jedoch soll in dieser Offenbarung der Begriff "Infusion" jede Verabreichung einer z.B. flüssigen oder gasförmigen oder festen Substanz bzw. eines Infusionsmittels, wie z.B. Medikamente, Zellen, Gene, Enzyme, Proteine, Antikörper, Hormone, Viren oder Ähnliches umfassen. Diese Substanzen werden meist direkt in einen Körper bzw. in Körpergewebe eingebracht, beispielsweise in das Gehirn eines Patienten. Das Zuführen der Substanz kann innerhalb relativ kurzer Zeit z.B. durch eine Injektion oder über einen längeren Zeitraum, beispielsweise bei kontinuierlicher oder gegebenenfalls variabler Zufuhrrate der Substanz erfolgen.

Aus der US 6,061,587 sind ein Verfahren und eine Vorrichtung zur gezielten Medikamentabgabe unter Verwendung von Magnetresonanz-Bilderfassung bekannt. Die US 5,583,902 offenbart ein Verfahren und eine Vorrichtung zur Vorhersage einer organspezifischen Kontrastverstärkung in einem Patienten vor einer Injektion. Die US 5,720,720 offenbart ein Verfahren zur Mikroinfusion mit hohen Strömungsraten, welches eine konvektionsverstärkte Abgabe von Mitteln in das Gehirn und andere feste Gewebestrukturen ermöglicht. Die US 5,205,289 beschreibt ein optimiertes Dosis-Verabreichungssystem unter Verwendung graphischer Simulationstechniken und computerunterstützter, numerischer Optimierung. Die US 3,690,318 offenbart eine Fluid-Infusionsvorrichtung mit variierbaren Strömungsregelungseinrichtungen. Aus der US 5,195,524 sind ein Verfahren und eine Vorrichtung zur Kernspin-Strömungsbilderfassung bekannt. Die US 5,840,026 beschreibt ein Kontrastmedium-Zuführungssystem, das die Einstellung der Kontrastmedium-Konzentration und der Injektionsparameter vor oder während einer Injektion startet.

Die Homogenität der Verteilung einer Infusion bzw. eines Infusionsfluids kann verschlechtert werden, wenn das Infusionsmittel in einen Bereich eingebracht wird, in welchem das Mittel durch gerichtete Kanäle transportiert wird, die selbst und deren Endpunkte nicht das eigentliche Infusionsziel sind. Anstatt in die eigentlichen Zielgebiete hinein zu diffundieren läuft das Infusionsmittel über solche "tracks" ab, ohne die gewünschte Wirkung zu erzielen.

Es ist die Aufgabe der vorliegenden Erfindung, eine erhöhte Kontrolle über die Infusionsmittelverteilung zu erzielen. Insbesondere soll verhindert werden, dass der Infusions-Einbringungsort zu nahe an Richtungskanäle gerät, in denen das Infusionsmittel schnell weitertransportiert wird und somit wirkungslos abläuft.

Diese Aufgabe wird gemäß einem Aspekt der vorliegenden Erfindung gelöst durch ein Verfahren zur Identifizierung von vorteilhaften bzw. nicht vorteilhaften Infusionsbereichen im Gewebe, bei dem funktionelle und/oder strukturelle Anatomiedaten erfasst werden, und bei dem computergestützt eine Auswertung der Anatomiedaten im Hinblick auf die darin enthaltenen Verteilungsinformationen, insbesondere Richtungsund/oder Geschwindigkeitsinformationen durchgeführt wird. Es können dadurch beispielsweise vor der Einbringung der Infusion die Richtungskanäle identifiziert, an denen bei einer Einbringung von Infusionsfluid mit einem schnellen Weitertransport zu rechnen ist. Grundlage hierfür bilden Anatomiedaten, wie sie beispielsweise durch ein bildgebendes System, wie einen Kernspintomographen, einen Computertumorgrafen oder ähnliche bekannte bildgebende, Systeme ermittelt werden können. Dabei ist es möglich, sowohl strukturelle Anatomiedaten, also lediglich Daten über den Gewebeaufbau zu erfassen, als auch funktionelle Informationen zu erhalten, beispielsweise Daten über gewisse Bereiche mit einer speziellen Funktion (Hör-Kortex, Seh-Kortex usw.) im Gehirn. Mit diesen Informationen lässt sich nun computergestützt ermitteln, welche Bereiche des Gewebes Transportwege beinhalten. Insbesondere lässt sich dabei auch herausfinden, ob bei der Einbringung an einer bestimmten Stelle ein wirkungsloser Ablauf des Infusionsmittels über sogenannte "tracks" erfolgen wird, oder ob eine homogene Diffusion in das umgebende Gewebe stattfindet. Vorteilhafterweise lassen sich damit also schon vor der Durchführung einer Infusion Zielgebiete mit vorteilhaften Verteilungseigenschaften von solchen mit weniger oder nicht vorteilhaften Verteilungseigenschaften unterscheiden.

Während der Infusion können sich die strukturellen und/oder anatomischen Eigenschaften des Gewebes verändern. Diese Veränderung kann sich beispielsweise durch die physikalischen und/oder biochemischen Gegebenheiten der Infusion selbst, aber auch durch eine Reaktion des infundierten Materials mit dem Gewebe ergeben. Um diese Veränderungen in ihrem Zeitlichen Verlauf zu berücksichtigen, können entsprechende Anpassungen der Verteilungsinformationen vorgenommen und dem Benutzer zu Verfügung gestellt werden.

Das Verfahren zur Identifikation von Transportwegen basiert auf Diffusionsmessungen. Solche Messungen werden in einem mathematischen Algorithmus durch Mittelwertbildung von Störsignalen bereinigt und zu richtungsungebundenen (gemittelten) oder auch zu richtungsgebundenen Geschwindigkeitsinformationen pro Volumenelement umgewandelt. Darstellungen der Isotropie bzw. Anisotropie können ebenfalls auf Diffusionsmessungen basieren und beinhalten Informationen über die Richtungsgebundenheit der Flüssigkeits-Durchlässigkeit (Permeabilität) eines Volumenelementes. Die Geschwindigkeits- und Isotropieinformationen können alleinstehend oder miteinander kombiniert weiterverwendet werden. Weiterhin können die Geschwindigkeits- und/oder Isotropieinformationen mit weiteren Anatomie-Daten kombiniert werden, um ihre Aussagekraft zu erhöhen oder zu spezifizieren.

Bei einer vorteilhaften Ausführungsform wird im Rahmen der Auswertung der Verteilungsinformationen die Geschwindigkeit der Diffusion eines Volumenelementes im Gewebe ermittelt, insbesondere werden Bereiche mit schneller Diffusion identifiziert.

Die Verteilungsinformationen, insbesondere Diffusionsgeschwindigkeit und Isotropie, können zweidimensional ermittelt werden, also auf der Basis zweidimensionaler Bildinformationen. Wenn mehrere solche zweidimensionale Bildinformations-Datensätze über die anatomische Struktur vorliegen, die in ihren Ebenen Informationen über die Infusionsfluidverteilung zugänglich machen, können diese zweidimensionalen Datensätze kombiniert werden, um dreidimensionale Verteilungsinformationen zu erhalten. Andererseits besteht natürlich die Möglichkeit, sogleich dreidimensionale Bilddatensätze zu ermitteln und hinsichtlich ihrer Verteilungsinformationen auszuwerten.

Gemäß einem weiteren Aspekt betrifft die Erfindung ein Verfahren zur Planungsunterstützung für die Einbringung eines Infusionsfluids, insbesondere in Gehirnbereichen, wobei vorteilhafte Infusionsbereiche durch ein Verfahren identifiziert werden, wie es oben beschrieben wurde. Ferner wird dabei noch die Einbringung der Infusion an einer ausgewählten Stelle geplant und/oder mittels einer medizinischen, vorzugsweise stereotaktischen Navigation ausgeführt. Demjenigen, der dann die Infusion setzt, kann mit Hilfe der Navigation aufgezeigt werden, wo das Ziel der Infusionsvorrichtung sein soll, und während des Setzens der Infusionsvorrichtung kann er angeleitet werden, die selbige soweit zu verschieben, bis das optimale Einbringungsziel erreicht ist. Dabei werden die Infusionsinstrumente durch ein Kamerasystem oder magnetisch durch bekannte Tracking-Verfahren verfolgt und ihre räumliche Lage wird in Beziehung zu der Patientenanatomie auf einer Bildausgabe dargestellt.

Es ist im Rahmen des vorgenannten Verfahrens möglich, anatomische, funktionelle und/oder strukturelle Gewebedaten mit Informationen über die zu erwartende Verteilung des Infusionsfluids zu kombinieren. Mit anderen Worten können die zur Verteilungssimulation ermittelten anatomischen Patientendaten natürlich auch bei der Navigation Verwendung finden, in dem sie im Navigationssystem referenziert bzw. registriert werden.

Gemäß einem weiteren Aspekt betrifft die vorliegende Erfindung eine Vorrichtung zur Planungsunterstützung für die Einbringung eines Infusionsfluids, insbesondere in Gehirnbereiche, mit einem bildgebenden Gerät, insbesondere einem Kernspintomographen zur Erfassung funktioneller und/oder struktureller Anatomiedaten, mit einem Computer, der eine Auswertung der Verteilungsinformationen eines Infusionsfluids bei Einbringung an bestimmten Stellen auf der Basis der erfassten Anatomiedaten erstellt, und mit einem computergestützten, medizinischen Planungs- und Navigationssystem zur Unterstützung der Positionierung einer Infusionsvorrichtung. Die Auswertung der Verteilungsinformationen und die Navigation können durch ein einziges Computersystem oder durch separate Computersysteme unterstützt werden. Eine solche Vorrichtung ermöglicht die Durchführung und Umsetzung der vorliegenden Erfindung mit den vorgenannten Vorteilen bezüglich der Infusionsmittelverteilung.

Das bildgebende Gerät, der bzw. die Computer und das Navigationssystem können über Datenverbindungen zum ständigen oder abrufbaren Datenaustausch miteinander verbunden sein. Es ist möglich, einzelne oder alle dieser Geräte in dieser Weise miteinander zu verbinden.

Ferner betrifft die Erfindung noch ein Programm, das, wenn es auf einem Computer läuft oder in einem Computer geladen ist, den Computer veranlasst, ein oben beschriebenes Verfahren durchzuführen, sowie ein Computerprogramm-Speichermedium, das ein solches Programm aufweist.

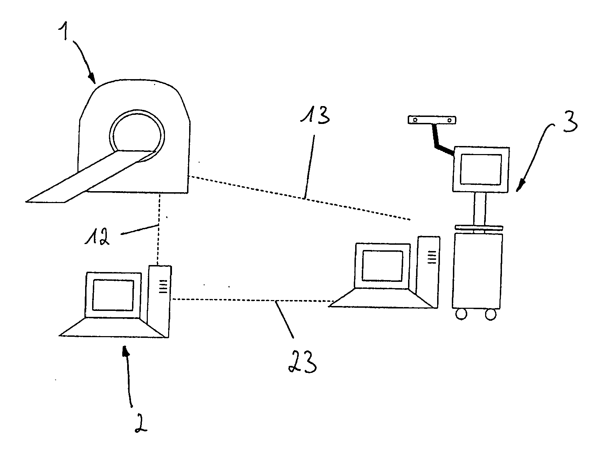

Die Erfindung wird anhand der beiliegenden Figur noch näher erläutert, welche schematisch eine Vorrichtung zur Realisierung der vorliegenden Erfindung darstellt. In der Zeichnung ist mit dem Bezugszeichen 1 ein bildgebendes Gerät dargestellt, hier ein Kernspintomograph mit einer nicht bezeichneten Patientenliege. Ein Patient wird beispielsweise mit dem Kopf in diesen Kernspintomograph eingebracht und es werden strukturelle und/oder funktionelle Anatomiedaten erfasst, durch die der Aufbau des Kopfes, insbesondere des Gehirns ermittelt wird. Nach der Verarbeitung der aufgenommenen Bilder, die schon in einem Prozessor im Kernspintomographen erfolgen kann, stehen nunmehr Informationen über den Aufbau des Gehirns des Patienten zur Verfügung. Bei einer vorteilhaften Ausführungsform werden diese Daten so aufgenommen, dass die Gehirnstruktur in einem medizinischen Navigationssystem registriert bzw. referenziert werden kann, wie es mit dem Bezugszeichen 3 angedeutet ist. Dass heißt, es werden vor der Kernspinaufnahme am Kopf des Patienten Markierungen angebracht, die sowohl in den Kernspinbildern, als auch bei einer Verfolgung durch das Navigationssystem identifiziert werden können, so dass das Positionsverhältnis zwischen den Markierungen und den erfassten Gehirnstrukturbereichen festgestellt wird und auch später im Laufe des navigierten Setzens der Infusion verwendet werden kann.

Zum Austausch der Daten zwischen dem Kernspintomographen 1 und dem Navigationssystem 3 steht eine Datenverbindung 13 zur Verfügung, die jegliche Form haben kann, also beispielweise eine Datenleitung, eine Funk-Datenübertragung oder eine Datenübertragung durch Übergabe von Speichermedien. Die Verbindung kann also eine on-line-(andauernde) Verbindung sein, sie kann aber auch eine auf Abruf verfügbare Verbindung sein (off-line).

Mit dem Bezugszeichen 2 ist schematisch ein Computersystem dargestellt, welches über die on-line- oder off-line-Verbindung 12 mit den Kernspintomographen 1 Daten austauschen kann. Die wie vorher beschreiben ermittelten funktionellen und/strukturellen Anatomiedaten aus dem Kernspintomographen 1 werden über die Leitung 12 dem Computer 2 mitgeteilt und in diesem Computer läuft nun ein Programm ab, welches auf der Basis dieser Anatomiedaten die Verteilungsinformationen auswertet und/oder anzeigt und/oder die Verteilung eines Infusionsfluids an bestimmten Stellen simuliert. Es wird sich dabei herausstellen, dass an einigen Stellen, an denen gerichtete und/oder schnelle Kanäle ("tracks") vorhanden sind, dann, wenn sie als Infusionspunkt ausgewählt werden, eine unvorteilhafte Verteilung erfolgen kann, nämlich ein schneller Ablauf in ungewünschte Gebiete. Andererseits werden Zielpunkte für die Infusion identifiziert, bei denen eine homogene Verteilung des Infusionsfluids zu erwarten ist. Der Computer 2 kann diese Stellen von den unvorteilhaften unterscheidungsfähig machen, also beispielsweise in erstellte Bilddatensätze entsprechende Kennzeichnungen einfügen.

Mit diesen Informationen über vorteilhafte und nicht vorteilhafte Infusionsbereiche im Gehirngewebe können nunmehr eine oder mehrere Positionen für die Infusionsvorrichtung vom Bediener so geplant werden, dass eine möglichst homogene Infusionsfluidverteilung zu erwarten ist. Hierzu gibt der Bediener des Computers 2 die gewünschten Positionen für die Infusionsvorrichtung mit Hilfe einer Benutzerschnittstelle unter Beachtung der ihm am Computer 2 präsentierten Informationen ein. Mit Hilfe der im Computer enthaltenen Verteilungsinformationen können durch den Computer auch vorteilhafte Zielpunkte für die Positionen der Infusionsvorrichtungen vorgeschlagen werden. Nach Abschluss dieses Ablaufes übergibt der Computer 2 die Positionen für die Infusionsvorrichtung und/oder weitere Informationen über die Schnittstelle 23 an das Navigationssystem 3. Dieses Navigationssystem kann ein bekanntes optisches Navigationssystem sein, wie es beispielsweise in der DE 196 396 152 beschrieben wird. Ein solches Navigationssystem 3, das den Patienten und medizinische Instrumente, beispielsweise die Infusionsrichtung, positionell verfolgt, registriert und in Referenz zu ermittelten Bilddaten auf einer Bildausgabe darstellt, ist als optisches, kameragestütztes System in der Zeichnung gezeigt. Es lassen sich aber auch andere Navigationssysteme einsetzen, beispielsweise magnetische oder induktive Navigationssysteme, die auf der Verfolgung von magnetischen bzw. induktiven Signalgebern im magnetischen bzw. elektrischen Feldern basieren.

Das Navigationssystem 3 wird beim Setzen der Infusion in der Nähe des Patienten aufgestellt bzw. der Patient wird dorthin gebracht. Das Navigationssystem 3 kennt die Anatomiedaten des Patienten, die über die Leitung 12 und/oder 23 mitgeteilt wurden, und es kann diese Anatomie räumlich zuordnen, da der Patient entsprechende Markierungen und/oder natürliche Landmarken trägt, die sowohl vom Kernspintomographen 1 lagemäßig aufgezeichnet wurden als auch durch das Navigationssystem über dessen Kamerasystem positionell erfasst werden können. Ferner kann das Navigationssystem eine mit entsprechenden Markierungen versehene Infusionsvorrichtung positionell verfolgen und so einbinden, dass für den Behandelnden ersichtlich wird, wo sich die Spitze der Infusionsvorrichtung gegenüber der Patientenanatomie aktuell befindet. Die dritte notwendige Information, nämlich die Daten über vorteilhafte bzw. nicht vorteilhafte Infusionsbereiche im Gewebe erhält das Navigationssystem über die Datenleitung 23 vom Computer 2 und mit Hilfe dieser Daten können im Gehirn spezielle Bereiche unterscheidungsfähig gemacht werden, die für den Infusions-Einbringungsort vorteilhaft sind oder nicht. Beim Setzen der Infusion kann nun auf der Bildausgabe des Navigationssystems 3 in zwei- oder dreidimensionaler Ansicht dargestellt werden, wo sich gerade die Spitze der Infusionsvorrichtung befindet und ob dieser Ort eine homogene Verteilung des Infusionsfluids verspricht oder nicht. Wenn der Arzt auf der Bildschirmausgabe erkennt, dass er mit der Spitze seines Instruments gerade einen vorteilhaften Infusionsbereich erreicht hat, kann das Instrument dort platziert werden.

Die Erfindung stellt deshalb ein Verfahren und eine Vorrichtung zur Verfügung, welche es verhindern, dass Infusionsöffnungen von Infusionsvorrichtungen an ungeeigneten Stellen im Gewebe zur Abgabe des Infusionsfluids platziert werden. Es steht eine erhöhte Kontrolle über die Wirksamkeit der Infusionsmittel-Verabreichung zur Verfügung; es kann also beispielsweise direkt und mit hoher Wirkung in bestimmte Tumore eingespritzt werden. Weil verhindert wird, dass das Infusionsmittel ohne Wirkung abläuft, ist es möglich, geringere Mengen des Infusionsmittels zu verwenden und wegen der ermöglichten homogenen Verteilung eine optimale Wirkung zu erzeugen. Ferner kann verhindert werden, dass andere Gehirnbereiche und/oder der Körper insgesamt durch unkontrolliert ablaufendes Infusionsfluid geschädigt werden.

## Patentansprüche

1. Verfahren zur Identifizierung von vorteilhaften bzw. nicht vorteilhaften Infusionsbereichen im Gewebe, bei dem funktionelle und strukturelle Anatomiedaten durch bildgebende Systeme erfasst werden, und bei dem computergestützt eine Auswertung der Anatomiedaten im Hinblick auf die darin enthaltenen Infusionsmittel-Verteilungsinformationen, nämlich Richtungs- und Geschwindigkeitsinformationen durchgeführt wird.

2. Verfahren nach Anspruch 1, bei dem die Auswertung der Anatomiedaten im Hinblick auf die darin enthaltenen Verteilungsinformationen zweidimensional erfolgt.

3. Verfahren nach Anspruch 1, bei dem die Auswertung der Anatomiedaten im Hinblick auf die darin enthaltenen Verteilungsinformationen dreidimensional erfolgt.

4. Verfahren nach einem der Ansprüche 1 bis 3, bei dem die Auswertung der Anatomiedaten im Hinblick auf die darin enthaltenen Verteilungsinformationen über einen Zeitraum hinweg erfolgt und eventuelle Anpassungen der Verteilungsinformationen an über die Zeit veränderte anatomische und/oder strukturelle Gegebenheiten vorgenommen werden.

5. Verfahren nach einem der Ansprüche 1 bis 4, bei dem die Geschwindigkeit der Diffusion des Infusionsfluids ermittelt wird, insbesondere Bereiche schneller Diffusion identifiziert werden.

6. Verfahren nach einem der Ansprüche 1 bis 5, bei dem die Isotropie und Anisotropie der Flussrichtungen im Gewebe ermittelt werden.

7. Verfahren nach einem der Ansprüche 1 bis 6, bei dem aus den funktionellen und/oder strukturellen Anatomiedaten das Verteilungsvolumen für ein Infusionsfluid berechnet wird.

8. Verfahren nach einem der Ansprüche 1 bis 7, bei dem die funktionellen und/oder strukturellen Anatomiedaten zweidimensional ermittelt werden.

9. Verfahren nach Anspruch 8, bei dem mehrere zweidimensionale Datensätze über die funktionellen und/oder strukturellen Anatomiedaten kombiniert werden, um dreidimensionale Informationen zu erhalten.

10. Verfahren nach einem der Ansprüche 1 bis 7, bei dem die funktionellen und/oder strukturellen Anatomiedaten dreidimensional ermittelt werden.

11. Verfahren zur Planungsunterstützung für die Einbringung eines Infusionsfluids, insbesondere in Gehirnbereiche, bei dem die Infusionsbereiche durch ein Verfahren nach einem der Ansprüche 1 bis 10 identifiziert werden, und bei dem mittels einer medizinischen, vorzugsweise stereotaktischen Planung die Einbringung der Infusion an einer ausgewählten Stelle geplant wird.

12. Verfahren zur Navigationsunterstützung für die Einbringung eines Infusionsfluids, insbesondere in Gehirnbereiche, bei dem die Infusionsbereiche und/oder Positionen für die Infusionsvorrichtung durch ein Verfahren nach einem der Ansprüche 1 bis 11 identifiziert werden, und bei dem mittels einer medizinischen, vorzugsweise stereotaktischen Navigation die Infusion und/oder die Infusionsvorrichtung an einer ausgewählten Stelle geplant wird.

13. Verfahren nach Anspruch 11 oder 12, bei dem zur Planung und/oder Navigation anatomische, funktionelle und/oder strukturelle Gewebedaten mit den Informationen über die zu erwartende Verteilung des Infusionsfluids kombiniert werden.

14. Vorrichtung zur Planungsunterstützung für die Einbringung eines Infusionsfluids, insbesondere in Gehirnbereiche, mit einem bildgebenden Gerät, insbesondere einem Kernspintomographen (1) zur Erfassung funktioneller und/oder struktureller Anatomiedaten, mit einem Computer (2), der eine Auswertung der funktionellen und/oder strukturellen Anatomiedaten zur Identifikation von vorteilhaften bzw. nicht vorteilhaften Infusionsbereichen vornimmt und/oder unterstützt, und/oder der die Simulation einer Verteilung eines Infusionsfluids bei Einbringung an bestimmten Stellen auf der Basis der erfassten Anatomiedaten erstellt und auswertet, und mit einem computergestützten, medizinischen Planungs- und Navigationssystem (3) zur Unterstützung der Positionierung einer Infusionsvorrichtung.

15. Vorrichtung nach Anspruch 14, bei der das bildgebende Gerät, der Computer (2) und das Navigationssystem über Datenverbindungen zum ständigen oder abrufbaren Datenaustausch miteinander verbunden sind.

16. Programm, das, wenn es auf einem Computer läuft oder in einem Computer geladen ist, den Computer veranlasst, ein Verfahren gemäß einem der Ansprüche 1 bis 13 durchzuführen.

17. Computerprogramm-Speichermedium, das ein Programm nach Anspruch 16 aufweist.

## Claims

1. A method for identifying advantageous and/or non-advantageous infusion regions in the tissue, wherein functional and structural anatomical data are captured by imaging systems, and wherein the anatomical data are evaluated with computer assistance, with respect to the infusion agent distribution information which they contain, i.e. directional and velocity information.

2. The method as set forth in claim 1, wherein the anatomical data are evaluated two-dimensionally with respect to the distribution information which they contain.

3. The method as set forth in claim 1, wherein the anatomical data are evaluated three-dimensionally with respect to the distribution information which they contain.

4. The method as set forth in any one of claims 1 to 3, wherein the anatomical data are evaluated over a period of time with respect to the distribution information which they contain and adjustments in the distribution information are possibly made to anatomical and/or structural conditions which have changed over the period of time.

5. The method as set forth in any one of claims 1 to 4, wherein the diffusion velocity of the infusion fluid is determined, in particular wherein regions of rapid diffusion are identified.

6. The method as set forth in any one of claims 1 to 5, wherein the isotropy and anisotropy of the flow directions in the tissue are determined.

7. The method as set forth in any one of claims 1 to 6, wherein the distribution volume for an infusion fluid is calculated from the functional and/or structural anatomical data.

8. The method as set forth in any one of claims 1 to 7, wherein the functional and/or structural anatomical data are determined two-dimensionally.

9. The method as set forth in claim 8, wherein a number of two-dimensional data sets on the functional and/or structural anatomical data are combined, to obtain three-dimensional information.

10. The method as set forth in any one of claims 1 to 7, wherein the functional and/or structural anatomical data are determined three-dimensionally.

11. A method for assisting planning for introducing an infusion fluid, in particular into regions of the brain, wherein the infusion regions are identified using a method as set forth in any one of claims 1 to 10, and wherein introducing the infusion at a selected point is planned by means of medical, preferably stereotactic planning.

12. A method for assisting navigation for introducing an infusion fluid, in particular into regions of the brain, wherein the infusion regions and/or positions for the infusion device are identified using a method as set forth in any one of claims 1 to 11, and wherein infusion and/or the introduction of the infusion device at a selected point is planned by means of medical, preferably stereotactic navigation.

13. The method as set forth in claim 11 or 12, wherein anatomical, functional and/or structural tissue data are combined with the information on the distribution of the infusion fluid to be expected, for planning and/or navigation.

14. A device for assisting planning for introducing an infusion fluid, in particular into regions of the brain, comprising: an imaging device, in particular a nuclear spin tomograph (1), for capturing functional and/or structural anatomical data; a computer (2) which performs and/or assists an evaluation of the functional and/or structural anatomical data, for identifying advantageous and/or non-advantageous infusion regions, and/or produces and evaluates a distribution simulation of an infusion fluid when it is introduced at particular points, on the basis of the captured anatomical data; and a computer-assisted, medical planning and navigation system (3) for assisting in positioning an infusion device.

15. The device as set forth in claim 14, wherein the imaging device, the computer (2) and the navigation system are connected to each other via data connections for a constant or retrievable exchange of data.

16. A program which, when run on a computer or loaded onto a computer, causes the computer to perform a method in accordance with any one of claims 1 to 13.

17. A computer program storage medium comprising a program as set forth in claim

## Revendications

1. Procédé pour identifier des zones d'infusion avantageuses et non avantageuses dans un tissu, dans lequel des données d'anatomie fonctionnelles et structurelles sont détectées par des systèmes générateurs d'image, et dans lequel une analyse des données d'anatomie est effectuée de manière assistée par ordinateur par rapport aux informations de distribution d'agent d'infusion contenues à l'intérieur, à savoir des informations de direction et de vitesse.

2. Procédé selon la revendication 1, dans lequel l'analyse des données d'anatomie par rapport aux informations de distribution contenues à l'intérieur s'effectue en deux dimensions.

3. Procédé selon la revendication 1, dans lequel l'analyse des données d'anatomie par rapport aux informations de distribution contenues à l'intérieur s'effectue en trois dimensions.

4. Procédé selon l'une quelconque des revendications 1 à 3, dans lequel l'analyse des données d'anatomie par rapport aux informations de distribution contenues à l'intérieur s'effectue sur une période, et d'éventuelles adaptations des informations de distribution à des données anatomiques et/ou structurelles, modifiées par rapport au temps, sont effectuées.

5. Procédé selon l'une quelconque des revendications 1 à 4, dans lequel la vitesse de diffusion du fluide d'infusion est déterminée, en particulier des zones de diffusion rapide sont identifiées.

6. Procédé selon l'une quelconque des revendications 1 à 5, dans lequel l'isotropie et l'anisotropie des directions de flux dans le tissu sont déterminées.

7. Procédé selon l'une quelconque des revendications 1 à 6, dans lequel le volume de distribution pour un fluide d'infusion est calculé à partir des données d'anatomie fonctionnelles et/ou structurelles.

8. Procédé selon l'une quelconque des revendications 1 à 7, dans lequel les données d'anatomie fonctionnelles et/ou structurelles sont déterminées en deux dimensions.

9. Procédé selon la revendication 8, dans lequel plusieurs ensembles de données en deux dimensions relatifs aux données d'anatomie fonctionnelles et/ou structurelles sont combinés, afin d'obtenir des informations en trois dimensions.

10. Procédé selon l'une quelconque des revendications 1 à 7, dans lequel les données d'anatomie fonctionnelles et/ou structurelles sont déterminées en trois dimensions.

11. Procédé pour l'assistance à la planification pour l'introduction d'un fluide d'infusion, en particulier dans des zones du cerveau, dans lequel les zones d'infusion sont identifiées par un procédé selon l'une quelconque des revendications 1 à 10, et dans lequel l'introduction de l'infusion en un endroit sélectionné est planifiée au moyen d'un planning médical, de préférence stéréotactique.

12. Procédé pour l'assistance à la navigation pour l'introduction d'un fluide d'infusion, en particulier dans des zones du cerveau, dans lequel les zones d'infusion et/ou positions pour le dispositif d'infusion sont identifiées par un procédé selon l'une quelconque des revendications 1 à 11, et dans lequel l'infusion et/ou le dispositif d'infusion en un endroit sélectionné est planifié au moyen d'une navigation médicale, de préférence stéréotactique.

13. Procédé selon la revendication 11 ou 12, dans lequel pour la planification et/ou la navigation des données de tissu anatomiques, fonctionnelles et/ou structurelles sont combinées avec les informations sur la distribution à escompter du fluide d'infusion.

14. Dispositif pour l'assistance à la planification pour l'introduction d'un fluide d'infusion, en particulier dans des zones du cerveau, comprenant un appareil générateur d'image, en particulier un tomographe à spin nucléaire (1) pour l'enregistrement de données d'anatomie fonctionnelles et/ou structurelles, avec un ordinateur (2), lequel effectue et/ou assiste une analyse des données d'anatomie fonctionnelles et/ou structurelles pour l'identification de zones d'infusion avantageuses ou non avantageuses, et/ou qui établit la simulation d'une distribution d'un fluide d'infusion lors de l'introduction en certains endroits sur la base des données d'anatomie enregistrées, et avec un système de planification et de navigation (3) médical assisté par ordinateur pour l'assistance au positionnement d'un dispositif d'infusion.

15. Dispositif selon la revendication 14, dans lequel l'appareil générateur d'image, l'ordinateur (2) et le système de navigation sont reliés par des liaisons de données pour un échange de données permanent ou interrogeable.

16. Programme qui, lorsqu'il fonctionne sur un ordinateur ou est chargé dans un ordinateur, permet à l'ordinateur de mettre en oeuvre un procédé selon l'une quelconque des revendications 1 à 13.

17. Support de mémoire de programme informatique, qui comporte un programme selon la revendication 16.
